# EUROPEAN PATENT SPECIFICATION

(11) **EP 4 444 238 B1**
(45) Date of publication and mention of the grant of the patent: **16.09.2026**
(21) Application number: 22826434.7
(22) Date of filing: 08.12.2022
(51) Int. Cl.: A61F 9/008

(54) **CORNEAL LENTICULAR INCISION USING A FEMTOSECOND LASER WITH PERIODIC LASER BLANKING IN CENTRAL AREA OF LENTICULE**
HORNHAUTLENTIKULÄRER EINSCHNITT MITHILFE EINES FEMTOSEKUNDEN-LASERS MIT PERIODISCHER LASERAUSTASTUNG IM ZENTRALEN BEREICH DES LENTIKELS
INCISION LENTICULAIRE CORNÉENNE UTILISANT UN LASER FEMTOSECONDE AVEC DÉCOUPAGE AU LASER PÉRIODIQUE DANS LA ZONE CENTRALE DU LENTICULE

(30) Priority: 10.12.2021 US 202117643812
(43) Date of publication of application: 16.10.2024
(73) Proprietor: AMO Development, LLC, Irvine, CA 92618 (US)
(72) Inventor: VILLANUEVA, Cynthia, Irvine, California 92618 (US); MEHTA-HURT, Deepali, Irvine, California 92618 (US); FU, Hong, Irvine, California 92618 (US); XU, Jiandong, San Jose, CA 95129 (US); MALEK TABRIZI, Alireza, Irvine, California 92618 (US); ALTMANN, Griffith, Ladera Ranch, California 92694 (US)
(74) Representative: Carpmaels & Ransford LLP
(86) International application number: PCT/IB2022/061912
(87) International publication number: WO 2023/105457

(56) References cited:
- US-A1- 2019 110 926
- US-A1- 2020 046 558

## Description

### BACKGROUND OF THE INVENTION

### Field of the Invention

Embodiments of this invention relate generally to laser-assisted ophthalmic procedures, and more particularly, to systems for lenticular incisions in the cornea.

### Description of Related Art

Vision impairments such as myopia (near-sightedness), hyperopia and astigmatism can be corrected using eyeglasses or contact lenses. Alternatively, the cornea of the eye can be reshaped surgically to provide the needed optical correction. Eye surgery has become commonplace with some patients pursuing it as an elective procedure to avoid using contact lenses or glasses to correct refractive problems, and others pursuing it to correct adverse conditions such as cataracts. And, with recent developments in laser technology, laser surgery is becoming the technique of choice for ophthalmic procedures.

Different laser eye surgical systems use different types of laser beams for the various procedures and indications. These include, for instance, ultraviolet lasers, infrared lasers, and near-infrared, ultra-short pulsed lasers. Ultra-short pulsed lasers emit radiation with pulse durations as short as 10 femtoseconds and as long as 3 nanoseconds, and a wavelength between 300 nm and 3000 nm.

Prior surgical approaches for reshaping the cornea include laser assisted in situ keratomileusis (hereinafter "LASIK"), photorefractive keratectomy (hereinafter "PRK") and corneal lenticule extraction.

In the LASIK procedure, an ultra-short pulsed laser is used to cut a corneal flap to expose the corneal stroma for photoablation with ultraviolet beams from an excimer laser. Photoablation of the corneal stroma reshapes the cornea and corrects the refractive condition such as myopia, hyperopia, astigmatism, and the like. In a PRK procedure where no flap is created, the epithelium layer is first removed, and some stroma material is then removed by an excimer laser. The epithelium layer will grow back within a few days after the procedure.

In a corneal lenticule extraction procedure, instead of ablating corneal tissue with an excimer laser following the creation of a corneal flap, the technique involves tissue removal with two or more femtosecond laser incisions that intersect to create a lenticule for extraction. The extraction of the lenticule changes the shape of the cornea and its optical power to accomplish vision correction. Lenticular extractions can be performed either with or without the creation of a corneal flap. With the flapless procedure, a refractive lenticule is created in the intact portion of the anterior cornea and removed through a small incision. Methods for corneal lenticule extraction using a fast-scan-slow-sweep scheme of a surgical ophthalmic laser system are described in U.S. Pat. Appl. Pub. No. 20160089270, entitled "Systems And Methods For Lenticular Laser Incision," published March 31, 2016, and U.S. Pat. Appl. Pub. No. 20200046558, entitled "High Speed Corneal Lenticular Incision Using A Femtosecond Laser," published February 13, 2020. Methods for corneal lenticule extraction using a fast-scan-slow-sweep scheme of a surgical ophthalmic laser system are also described in US2019110926A1.

### SUMMARY OF THE INVENTION

The invention is defined by the appended independent claims. Certain embodiments are defined by the appended dependent claims.

In one aspect, embodiments of the present invention provides an ophthalmic surgical laser system which includes: a laser source configured to generate a pulsed laser beam comprising a plurality of laser pulses; a laser delivery system configured to deliver the pulsed laser beam to a target tissue in a subject's eye; a high frequency scanner configured to scan the pulsed laser beam back and forth at a predefined frequency; an XY-scanner configured to deflect the pulsed laser beam, the XY-scanner being separate from the high frequency scanner; a Z-scanner configured to modify a depth of a focus of the pulsed laser beam; and a controller configured to control the laser source, the high frequency scanner, the XY-scanner and the Z-scanner to successively form a plurality of sweeps which collectively form at least one lenticular incision of a lens in the subject's eye, the lens having a curved surface that defines an apex and a Z axis passing through the apex, wherein each sweep is formed by: controlling the high frequency scanner to deflect the pulsed laser beam to form a scan line, the scan line being a straight line having a predefined length and being tangential to a parallel of latitude of the lens, the parallel of latitude being a circle on the surface of the lens that is perpendicular to the Z axis and has a defined distance to the apex, controlling the XY-scanner and the Z-scanner to move the scan line along a meridian of longitude of the lens, the meridian of longitude being a curve that passes through the apex and has a defined angular position around the Z axis, and controlling the laser source to periodically blank the pulsed laser beam when the scan line is located within a central area of the lens by periodically blanking the pulsed laser beam with a duty cycle of 5-95% and a period of 1.0-50.0 ms, wherein the plurality of sweeps are successively formed one after another along the respective meridians of longitude which are different from one another.

In another aspect, embodiments of the present invention provide a computer program product to operate a laser surgical system to perform a method for creating a lenticular incision, the method including the steps of: generating, by a laser source, a pulsed laser beam comprising a plurality of laser pulses; delivering the pulsed laser beam to a target tissue in a subject's eye; scanning, by a high frequency scanner, the pulsed laser beam back and forth at a predefined frequency; deflecting, by an XY-scanner, the pulsed laser beam, the XY-scanner being separate from the high frequency scanner; modifying, by a Z-scanner, a depth of a focus of the pulsed laser beam; and controlling, by a controller, the laser source, the high frequency scanner, the XY-scanner and the Z-scanner to successively form a plurality of sweeps which collectively form at least one lenticular incision of a lens in the subject's eye, the lens having a curved surface that defines an apex and a Z axis passing through the apex, including forming each sweep by: controlling the high frequency scanner to deflect the pulsed laser beam to form a scan line, the scan line being a straight line having a predefined length and being tangential to a parallel of latitude of the lens, the parallel of latitude being a circle on the surface of the lens that is perpendicular to the Z axis and has a defined distance to the apex, controlling the XY-scanner and the Z-scanner to move the scan line along a meridian of longitude of the lens, the meridian of longitude being a curve that passes through the apex and has a defined angular position around the Z axis, and controlling the laser source to periodically blank the pulsed laser beam when the scan line is located within a central area of the lens by periodically blanking the pulsed laser beam with a duty cycle of 5-95% and a period of 1.0-50.0 ms, wherein the plurality of sweeps are successively formed one after another along the respective meridians of longitude which are different from one another.

This summary and the following detailed description are merely exemplary, illustrative, and explanatory, and are not intended to limit, but to provide further explanation of the invention as claimed. Additional features and advantages of the invention will be set forth in the descriptions that follow, and in part will be apparent from the description, or may be learned by practice of the invention. The objectives and other advantages of the invention will be realized and attained by the structure particularly pointed out in the written description, claims and the appended drawings.

### BRIEF DESCRIPTION OF THE DRAWINGS

The novel features of the invention are set forth with particularity in the appended claims. A better understanding of the features and advantages will be facilitated by referring to the following detailed description that sets forth illustrative embodiments using principles of the invention, as well as to the accompanying drawings, in which like numerals refer to like parts throughout the different views. Like parts, however, do not always have like reference numerals. Further, the drawings are not drawn to scale, and emphasis has instead been placed on illustrating the principles of the invention. All illustrations are intended to convey concepts, where relative sizes, shapes, and other detailed attributes may be illustrated schematically rather than depicted literally or precisely.
FIG. 1 is a perspective view of a surgical ophthalmic laser system according to an embodiment of the present invention which may be used to perform a lenticule incision method.
FIG. 2 is another perspective view of a surgical ophthalmic laser system according to an embodiment of the present invention which may be used to perform a lenticule incision method.
FIG. 3 is a simplified diagram of a controller of a surgical ophthalmic laser system according to an embodiment of the present invention which may be used to perform a lenticule incision method.
FIG. 4 illustrates an exemplary scanning of a surgical ophthalmic laser system according to an embodiment of the present invention.
FIG. 5 illustrates an exemplary surface dissection using a fast-scan-slow-sweep scheme of a surgical ophthalmic laser system according to an embodiment of the present invention.
FIG. 6 illustrates a geometric relation between a fast-scan line and an intended spherical dissection surface of a surgical ophthalmic laser system according to an embodiment of the present invention.
FIG. 7 illustrates an exemplary lenticular incision using a surgical ophthalmic laser system according to an embodiment of the present invention.
FIG. 8 schematically illustrates a method for lenticule incision using a fast-scan-slow-sweep scheme with periodic laser blanking in the central area of the lenticule according to the operation of an embodiment of the present invention.
FIG. 9 schematically illustrates the laser blanking control signal for the periodic laser blanking in the method of Fig. 8.
FIG. 10 shows a table that summarizes laser blanking control parameters according to embodiments of the present invention.
FIG. 11 is a flowchart illustrating a lenticule incision process.

### DETAILED DESCRIPTION OF PREFERRED EMBODIMENTS

Embodiments of this invention are generally directed to systems for laser-assisted ophthalmic procedures, and more particularly, to systems for corneal lenticule incision.

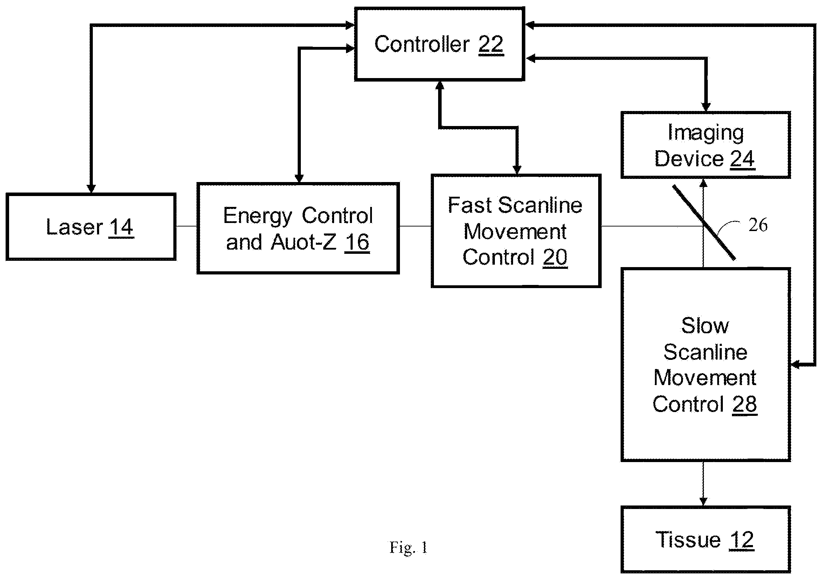

Referring to the drawings, FIG. 1 shows a system 10 for making an incision in a tissue 12 of a patient's eye. The system 10 includes, but is not limited to, a laser 14 capable of generating a pulsed laser beam, an energy control module 16 for varying the pulse energy of the pulsed laser beam, a fast scanline movement control module 20 for generating a fast scanline of the pulsed laser beam (described in more detail later), a controller 22, and a slow scanline movement control module 28 for moving the laser scanline and delivering it to the tissue 12. The controller 22, such as a processor operating suitable control software, is operatively coupled with the fast scanline movement control module 20, the slow scanline movement control module 28, and the energy control module 16 to direct the scanline of the pulsed laser beam along a scan pattern on or in the tissue 12. In this embodiment, the system 10 further includes a beam splitter 26 and a imaging device 24 coupled to the controller 22 for a feedback control mechanism (not shown) of the pulsed laser beam. Other feedback methods may also be used. In an embodiment, the pattern of pulses may be summarized in machine readable data of tangible storage media in the form of a treatment table. The treatment table may be adjusted according to feedback input into the controller 22 from an automated image analysis system in response to feedback data provided from a monitoring system feedback system (not shown).

Laser 14 may comprise a femtosecond laser capable of providing pulsed laser beams, which may be used in optical procedures, such as localized photodisruption (e.g., laser induced optical breakdown). Localized photodisruptions can be placed at or below the surface of the tissue or other material to produce high-precision material processing. For example, a micro-optics scanning system may be used to scan the pulsed laser beam to produce an incision in the material, create a flap of the material, create a pocket within the material, form removable structures of the material, and the like. The term "scan" or "scanning" refers to the movement of the focal point of the pulsed laser beam along a desired path or in a desired pattern.

In other embodiments, the laser 14 may comprise a laser source configured to deliver an ultraviolet laser beam comprising a plurality of ultraviolet laser pulses capable of photodecomposing one or more intraocular targets within the eye.

Although the laser system 10 may be used to photoalter a variety of materials (e.g., organic, inorganic, or a combination thereof), the laser system 10 is suitable for ophthalmic applications in some embodiments. In these cases, the focusing optics direct the pulsed laser beam toward an eye (for example, onto or into a cornea) for plasma mediated (for example, non-UV) photoablation of superficial tissue, or into the stroma of the cornea for intrastromal photodisruption of tissue. In these embodiments, the surgical laser system 10 may also include a lens to change the shape (for example, flatten or curve) of the cornea prior to scanning the pulsed laser beam toward the eye.

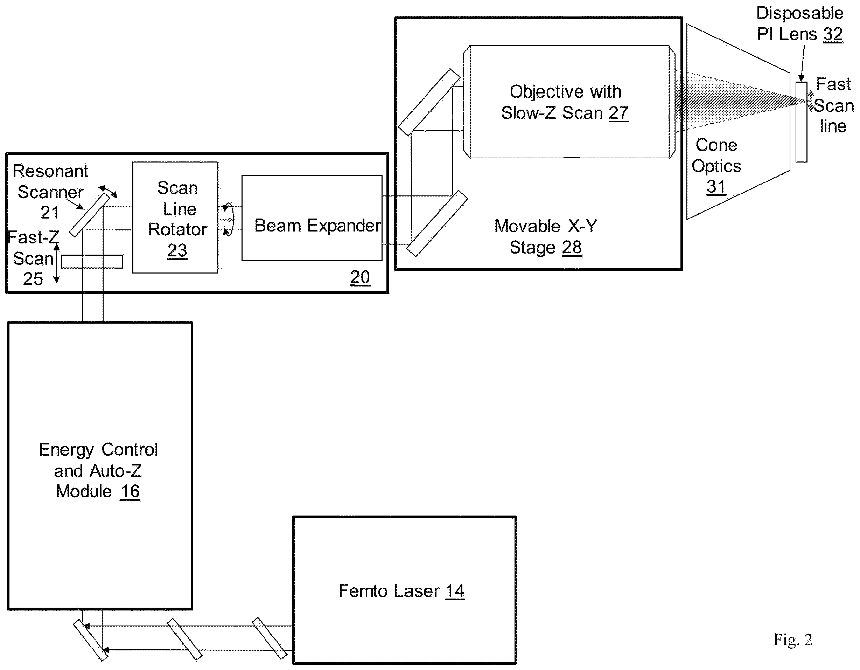

FIG. 2 shows another exemplary diagram of the laser system 10. FIG. 2 shows components of a laser delivery system including a moveable XY-scanner (or movable XY-stage) 28 of a miniaturized femtosecond laser system. In this embodiment, the system 10 uses a femtosecond oscillator, or a fiber oscillator-based low energy laser. This allows the laser to be made much smaller. The laser-tissue interaction is in the low-density-plasma mode. An exemplary set of laser parameters for such lasers include pulse energy in the 40-100 nJ range and pulse repetitive rates (or "rep rates") in the 2-40 MHz range. A fast-Z scanner 25 and a resonant scanner 21 direct the laser beam to a scanline rotator 23. When used in an ophthalmic procedure, the system 10 also includes a patient interface design that has a fixed cone nose 31 and a contact lens 32 that engages with the patient's eye. A beam splitter may be placed inside the cone 31 of the patient interface to allow the whole eye to be imaged via visualization optics. In some embodiments, the system 10 may use: optics with a 0.6 numerical aperture (NA) which would produce 1.1 µm Full Width at Half Maximum (FWHM) focus spot size; and a resonant scanner 21 that produces 0.2-1.2 mm scan line with the XY-scanner scanning the resonant scan line to a 1.0 mm field. The prism 23 (e.g., a Dove or Pechan prism, or the like) rotates the resonant scan line in any direction on the XY plane. The fast-Z scanner 25 sets the incision depth. The slow scanline movement control module employs a movable XY-stage 28 carrying an objective lens with Z-scanning capability 27, referred to as slow-Z scanner because it is slower than the fast-Z scanner 25. The movable XY-stage 28 moves the objective lens to achieve scanning of the laser scanline in the X and Y directions. The objective lens changes the depth of the laser scanline in the tissue. The energy control and auto-Z module 16 may include appropriate components to control the laser pulse energy, including attenuators, etc. It may also include an auto-Z module which employs a confocal or non-confocal imaging system to provide a depth reference. The miniaturized femtosecond laser system 10 may be a desktop system so that the patient sits upright while being under treatment. This eliminates the need of certain opto-mechanical arm mechanism(s), and greatly reduces the complexity, size, and weight of the laser system. Alternatively, the miniaturized laser system may be designed as a conventional femtosecond laser system, where the patient is treated while lying down.

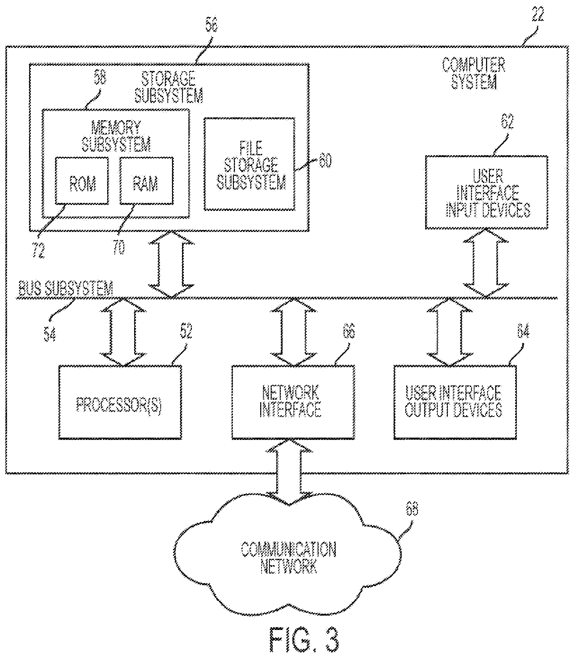

FIG. 3 illustrates a simplified block diagram of an exemplary controller 22 that may be used by the laser system 10 according to an embodiment of this invention to control the laser system 10 and execute at least some of the steps discussed in detail below. Controller 22 typically includes at least one processor 52 which may communicate with a number of peripheral devices via a bus subsystem 54. These peripheral devices may include a storage subsystem 56, comprising a memory subsystem 58 and a file storage subsystem 60, user interface input devices 62, user interface output devices 64, and a network interface subsystem 66. Network interface subsystem 66 provides an interface to outside networks 68 and/or other devices. Network interface subsystem 66 includes one or more interfaces known in the arts, such as LAN, WLAN, Bluetooth, other wire and wireless interfaces, and so on.

User interface input devices 62 may include a keyboard, pointing devices such as a mouse, trackball, touch pad, or graphics tablet, a scanner, foot pedals, a joystick, a touch screen incorporated into a display, audio input devices such as voice recognition systems, microphones, and other types of input devices. In general, the term "input device" is intended to include a variety of conventional and proprietary devices and ways to input information into controller 22.

User interface output devices 64 may include a display subsystem, a printer, a fax machine, or non-visual displays such as audio output devices. The display subsystem may be a flat-panel device such as a liquid crystal display (LCD), a light emitting diode (LED) display, a touchscreen display, or the like. The display subsystem may also provide a non-visual display such as via audio output devices. In general, the term "output device" is intended to include a variety of conventional and proprietary devices and ways to output information from controller 22 to a user.

Storage subsystem 56 can store the basic programming and data constructs that provide the functionality of the various embodiments of the present invention. For example, a database and modules implementing the functionality of the methods of the present invention, as described herein, may be stored in storage subsystem 56. These software modules are generally executed by processor 52. In a distributed environment, the software modules may be stored on a plurality of computer systems and executed by processors of the plurality of computer systems. Storage subsystem 56 typically comprises memory subsystem 58 and file storage subsystem 60.

Memory subsystem 58 typically includes a number of memories including a main random access memory (RAM) 70 for storage of instructions and data during program execution and a read only memory (ROM) 72 in which fixed instructions are stored. File storage subsystem 60 provides persistent (non-volatile) storage for program and data files. File storage subsystem 60 may include a hard disk drive along with associated removable media, a Compact Disk (CD) drive, an optical drive, DVD, solid-state memory, and/or other removable media. One or more of the drives may be located at remote locations on other connected computers at other sites coupled to controller 22. The modules implementing the functionality of the present invention may be stored by file storage subsystem 60.

Bus subsystem 54 provides a mechanism for letting the various components and subsystems of controller 22 communicate with each other as intended. The various subsystems and components of controller 22 need not be at the same physical location but may be distributed at various locations within a distributed network. Although bus subsystem 54 is shown schematically as a single bus, alternate embodiments of the bus subsystem may utilize multiple busses.

Due to the ever-changing nature of computers and networks, the description of controller 22 depicted in FIG. 3 is intended only as an example for purposes of illustrating only one embodiment of the present invention. Many other configurations of controller 22, having more or fewer components than those depicted in FIG. 3, are possible.

As should be understood by those of skill in the art, additional components and subsystems may be included with laser system 10. For example, spatial and/or temporal integrators may be included to control the distribution of energy within the laser beam. Ablation effluent evacuators/filters, aspirators, and other ancillary components of the surgical laser system are known in the art, and may be included in the system. In addition, an imaging device or system may be used to guide the laser beam.

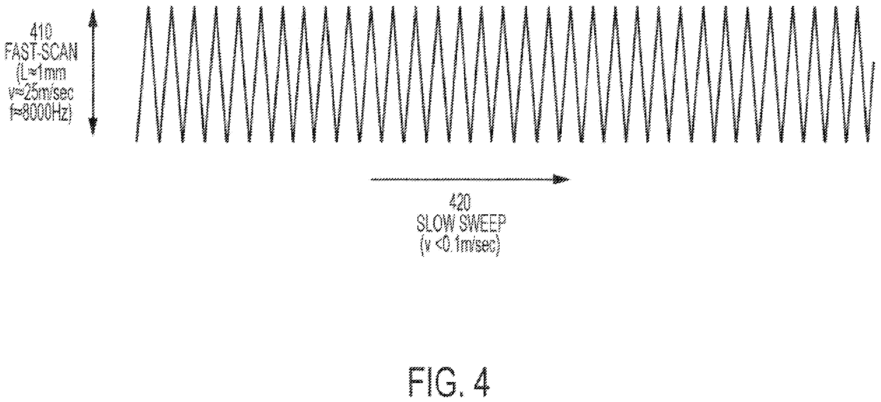

In preferred embodiments, the beam scanning can be realized with a "fast-scan-slow-sweep" scanning scheme, also referred herein as a fast-scan line scheme. The scheme consists of two scanning mechanisms: first, a high frequency fast scanner is used to scan the beam back and forth to produce a short, fast scan line (e.g., a resonant scanner 21 of FIG. 2); second, the fast scan line is slowly swept by much slower X, Y, and Z scan mechanisms (e.g. the moveable X-Y stage 28 and the objective lens with slow-Z scan 27, and the fast-Z scanner 25). FIG. 4 illustrates a scanning example of a laser system 10 using an 8 kHz (e.g. between 7 kHz and 9 kHz, or more generally, between 0.5 kHz and 20 kHz) resonant scanner 21 to produce a fast scan line 410 of about 1 mm (e.g., between 0.9 mm and 1.1 mm, or more generally, between 0.2 mm and 1.2 mm) and a scan speed of about 25 m/sec, and X, Y, and Z scan mechanisms with the scan speed (sweeping speed) smaller than about 0.1 m/sec. The fast scan line 410 may be perpendicular to the optical beam propagation direction, i.e., it is always parallel to the XY plane. The trajectory of the slow sweep 420 can be any three dimensional curve drawn by the X, Y, and Z scanning devices (e.g., XY-scanner 28 and fast-Z scanner 25). An advantage of the "fast-scan-slow-sweep" scanning scheme is that it only uses small field optics (e.g., a field diameter of 1.5 mm) which can achieve high focus quality at relatively low cost. The large surgical field (e.g., a field diameter of 10 mm or greater) is achieved with the XY-scanner, which may be unlimited.

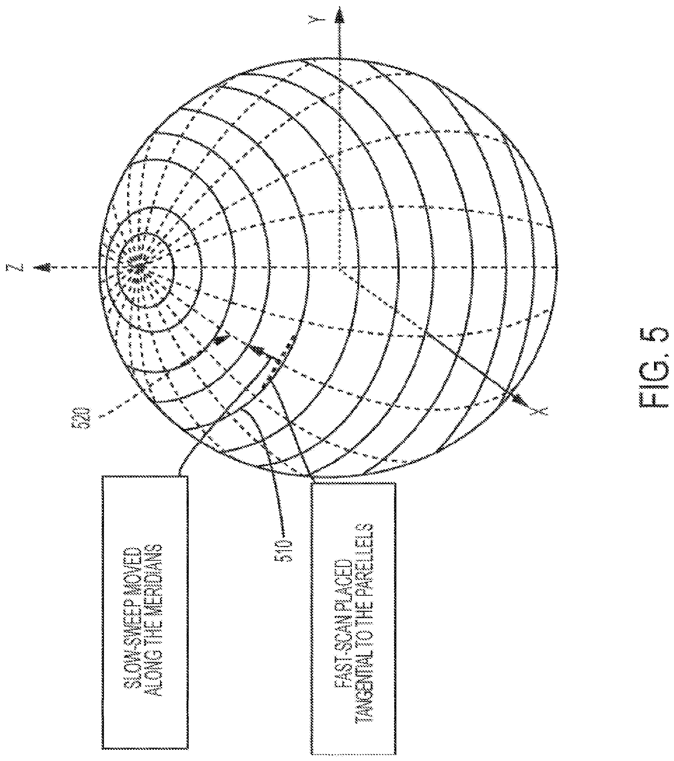

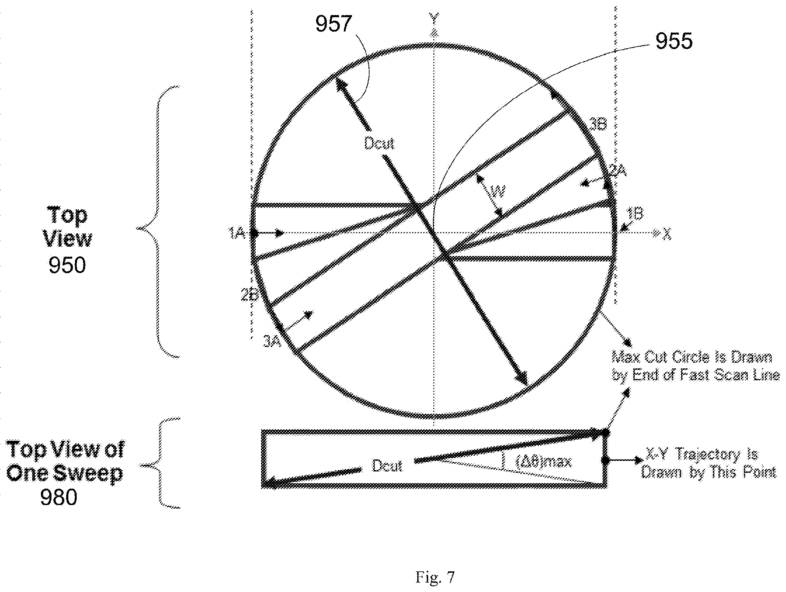

In a preferred embodiment shown in FIG. 5 and 7A-7B, the laser system 10 creates a smooth lenticular cut using the "fast-scan-slow-sweep" scanning scheme under a preferred procedure. First, in a three dimensional lenticular cut, the fast scan line is preferably placed tangential to the parallels of latitude 510 on the surface of the lenticule. A parallel of latitude is the intersection of the surface with a plane perpendicular to the Z axis (which is the axis parallel to the depth direction of the eye), i.e. a circle on the surface of the lens that is perpendicular to the Z axis and has a defined distance to the apex (the highest point in the Z direction). For example, in the laser system 10 of FIG. 2, this can be realized by adjusting a prism 23 to the corresponding orientations via software, e.g., via the controller 22. Second, the slow sweep trajectory preferably moves along the meridians of longitude 520 on the surface of the lenticule. A meridian of longitude is the intersection of the surface with a plane that passes through the Z axis, i.e. a curve that passes through the apex and has a defined angular direction with respect to the Z axis. For example, in the laser system of FIG. 2, this can be done by coordinating the XY scanner 28, and the Fast-Z scanner 25 via the software, e.g., via the controller 22. The procedure starts with the scan line being parallel to the XY plane, and sweeps through the apex of the lens, following the curvature with the largest diameter (see also FIG. 7A). Multiple sweeps are performed at successive angular directions with respect to the Z axis, for example as realized by rotating the prism 23 between successive sweeps, to form the entire lenticule. With this preferred procedure, there are no vertical "steps" in the dissection, and vertical side cuts are eliminated. As will be analyzed herein below, the deviations between the laser focus locations and the intended spherical surface dissections are also minimized.

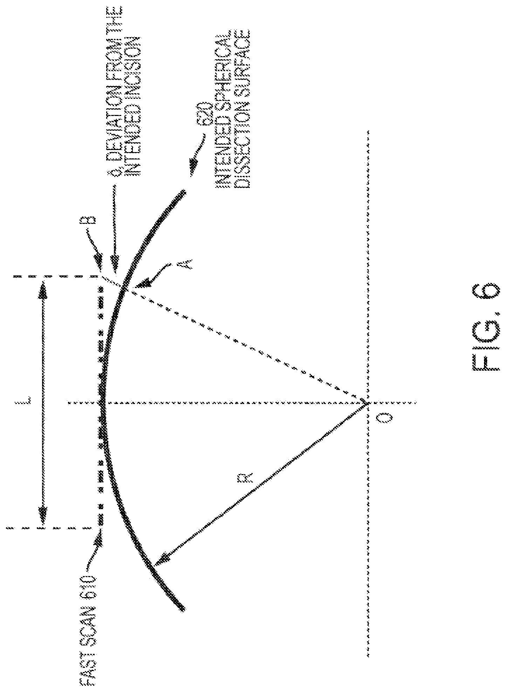

FIG. 6 shows the geometric relation between the fast scan line 610 and the intended spherical dissection surface 620, e.g., of a lens, especially the distance deviation (δ) between the end point B of the scan line 610 and point A on the intended dissection surface 620. The maximum deviation δ is the distance between point A and point B, and is given by (Equation (1)): $δ = \sqrt{{R}^{2} + \frac{{L}^{2}}{4}} - R \approx \frac{{L}^{2}}{8 R}$ where R is greater than L. R is the radius of curvature of the surface dissection 620, and L is the length of the fast scan.

While the above maximum deviation analysis is for a spherical surface, this scanning method may also be used to create a smooth cut having a non-spherical shape , such as an ellipsoidal shape, etc. In such a case, the parallel of latitude and/or the meridian of longitude may not be circular.

In an exemplary case of myopic correction, the radius of curvature of the surface dissection may be determined by the amount of correction, ΔD, using the following equation (Equation (2)): $Δ D = \frac{\left(n-1\right)}{{R}_{1}} + \frac{\left(n-1\right)}{{R}_{2}}$ where n=1.376, which is the refractive index of cornea, and *R₁* and *R₂* (may also be referred herein as *Rₜ* and *R_{b}*) are the radii of curvature for the top surface and bottom surface of a lenticular incision, respectively. For a lenticular incision with *R₁=R₂=R* (the two dissection surface are equal for them to physically match and be in contact), we have (Equation (3)): $R = \frac{2 \left(n-1\right)}{Δ D}$

FIG. 7 is a top view 950 of a lenticular incision 900 which illustrates three exemplary sweeps (1A to 1B), (2A to 2B) and (3A to 3B), with each sweep going through (i.e., going over) the lenticular incision apex 955. The incision diameter 957 (D_{CUT}) should be equal to or greater than the to-be-extracted lenticular incision diameter. A top view 980 shows the top view of one exemplary sweep.

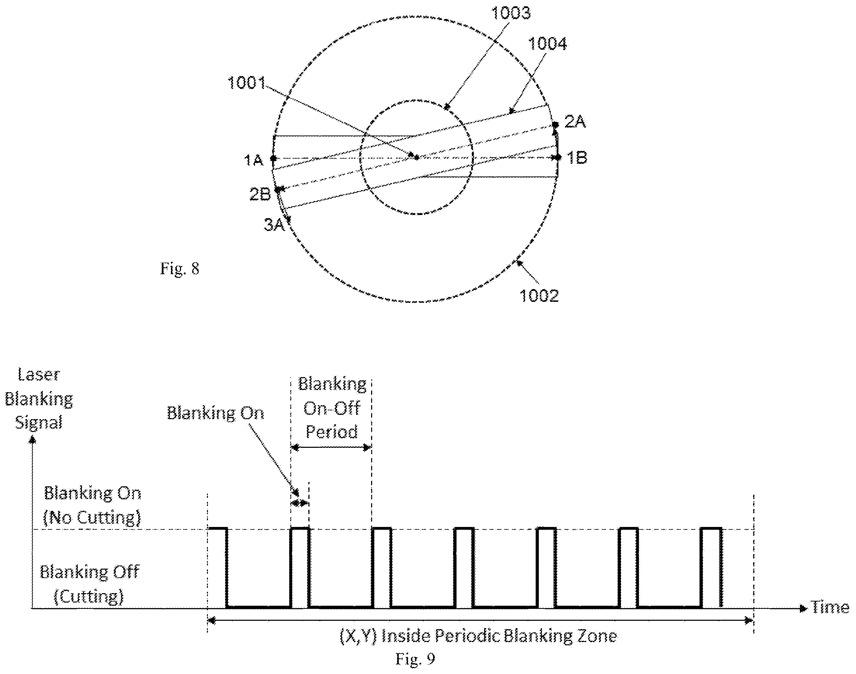

Using such a "fast-scan-slow-sweep" scanning scheme, each sweep of the fast scan line forms a bent band, the bent band being the equivalent of bending a flat rectangle such that its long sides form arched shapes (the shape of the meridian of longitude) while its short sides remain straight. In the top view in FIG. 7 and FIG. 8, the rectangular shapes represent the sweeps. In the central area of the lenticule cut, i.e. the area closer to the apex, multiple sweeps overlap each other. The amount of overlap decreases toward the edge of the lenticule cut. The inventors recognized that when uniform sweeps are used, the central area experiences significant redundant cutting, causing unnecessary high energy deposit in this area. This is disadvantages because it may cause unnecessary cavitation bubbles which in turn may cause light scattering induced glare and halo. In particular, the high energy area is located at the center of the visual field, making it even more undesirable. The excessive bubbles at the lenticule center may cause displacement of the tissue during cutting, such as producing a center hole when the lenticule is thin; it may also result in a relatively thick lenticule cutting interface.

U.S. Pat. Appl. Pub. No. 20200046558 describes corneal lenticule incision method which addresses this redundant cutting problem by using a variable sweeping speed along the meridian, so that in each sweep, the sweeping speed is the slowest at the edge of the lenticule and the fastest near the apex.

Preferred embodiments of the present invention address the redundant cutting problem by applying rapid laser blanking in the central area of the lenticule. This technique maintains the desired tissue-bridge free cutting performance, and effectively reduces the excessive bubbles generated during lenticule incisions.

More specifically, in preferred embodiments of the present invention, the laser is periodically blanked in the central area of the lenticule during each sweep. The generation of the fast scan lines is unchanged, and the sweeping speed for each sweep may be constant or variable. As schematically illustrated in Fig. 8 (top view of the lenticule), the laser blanking zone 1003 is a central area (circular in this example) of the lenticule within the lenticule boundary 1002, centered at the apex (lenticule center) 1001. During each sweep 1004, which starts from the edge of the lenticule and proceeds along a meridian to the opposite part of the edge, when the center position of the fast scan line is inside the blanking zone 1003, the laser is blanked periodically; outside the blanking zone 1003, the laser is not blanked.

As shown in Fig. 9, inside the blanking zone 1003, there are a number of blanking on-off periods; within each blanking on-off period, the laser blanking signal is On (i.e., the laser is blanked) for a fraction of the period, and Off (i.e., the laser is not blanked) for the rest of the period. This reduces the total number of laser pulses delivered in the blanking zone 1003, which reduces the amount of redundant cutting. Outside the blanking zone 1003, the laser blanking signal is Off.

The periodic laser blanking scheme is accomplished by the ophthalmic laser system under the control of the controller, based on the following control parameters:
(1) Laser blanking Enable or Disable. When this parameter is disabled, no blanking is performed.
(2) Laser blanking zone diameter, e.g. in mm.
(3) Laser blanking On-Off period, e.g. in ms.
(4) Laser blanking duty cycle, i.e., the percentage of time within each laser blanking On-Off period that laser blanking is On.

These parameters, along with other parameters of the laser system, determine the amount of laser energy reduction in the central area of the lenticule incision.

In one particular example, the resonant scanner frequency is 7910 Hz; i.e.,. the system generates 15820 scan lines per second, each scan line being 63 µs (0.063 ms) in duration, or about 16 scan lines per ms. The laser repetition rate is 10M pulses per second, so each scan line has approximately 632 laser pulses. The laser blanking On-Off mode can be switched within nanoseconds, but the laser pulse behavior transient time is about 30 µs (0.03 ms); thus, for each On-Off transition, about half scan line will be in this transient time and will therefore cut poorly. Near the lenticule center, the XY scanning speed is about 32-40 mm/s, or more generally, about 10-100 mm/s. When the blanking zone diameter is 1.5 mm, the time inside the blanking zone in each sweep is about 40 ms, i.e., about 630 scan lines inside the zone. When the blanking On-Off period is 1 ms and the blanking duty cycle is 5%, each laser blanking signal On time duration is about 0.05 ms (50 µs). Taking into consideration the transient time mentioned above, the total time during which adequate cutting does not occur (the uncut time) is about 80 µs. As a result, in this example, for every 16 scan lines, there will be 1-1.5 uncut line due to laser blanking.

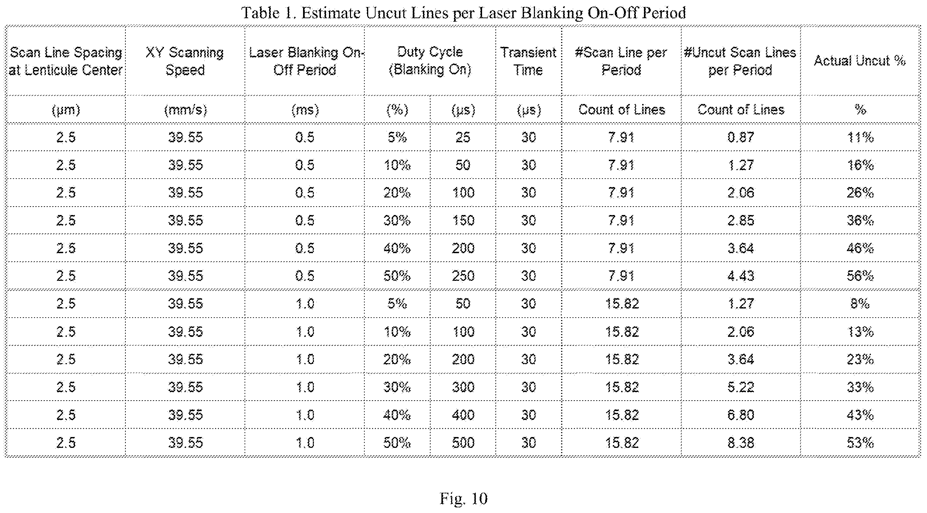

The parameters for additional examples are shown in Table 1 in Fig. 10, giving the percentage of actual uncut time in each example.

More generally, for the periodic laser blanking method, the blanking duty cycle is 5-95%, and may preferably be 15-25%, and more preferably 20%; the blanking On-Off period is 1-50 ms, and may preferably be 2.5-7.5 ms, and more preferably 5 ms. When the period is 5 ms and the duty cycle is 20%, the actual blanking time per period is about 1 ms. In a preferred embodiment, each 1 ms corresponds to approximately 32 µm of uncut length of the sweep. Generally speaking, uncut length should not be too long, e.g., longer than 32 µm. The approximately 1 ms uncut time (approximately 32 µm uncut length) gives more evenly distributed cut-uncut-cut-uncut regions. This will reduce or eliminate tissue bridges. The resulting percentage of actual uncut time may be 5 to 95%, preferably 10 to 30%, more preferable, 20%; the blanking zone radius may be 0.25-2.5 mm, preferably 0.5-1.0 mm, more preferably 0.75 mm.

The blanking of laser pulses in a high-repetition-rate laser system may be achieved in various ways. For example, a pulse picker (e.g., an acoustic-optical modulator, AOM) may be used to selectively pick some laser pulses and block other laser pulses. To maintain beam quality and avoid wavefront aberration, the pulse picking is done before light amplification. However, simply blocking the pulses before the amplifier of the laser system may create a problem of "giant pulses." Namely, if in the blanking-on time period, there is no laser pulse passing through the amplifier, then the first laser pulse in the blanking-off time period immediately following the blanking-on time period will experience extra gain when it passes through the amplifier and will become a "giant pulse." This is undesirable because giant pulses may cause abnormally large tissue effect.

Thus, in preferred embodiments of the present invention, the laser pulses are not blocked before the amplifier; rather, in the blanking-on time period, the laser is switched to a higher repetition rate, lower pulse energy mode, to generate laser pulses at a higher pulse repetition rate but a lower pulse energy. The lower pulse energy is such that at the locations they are delivered to the tissue, the pulse energy is below the tissue's photodisruption threshold (the energy at which the a laser pulse starts to photodisrupt the tissue), and therefore will not result in any tissue cutting. Meanwhile, because these more numerous lower pulse energy pulses pass through the amplifier, the amplifier is not in an idle state during the blanking-on period and therefore, it will not generate the "giant pulse" in the blanking-off period. This method can realize fast blanking for the laser incision procedures. In one particular example, a 10 MHz repetition rate is used to perform normal laser cutting, and laser blanking is realized by switching from the 10 MHz to a 40 MHz repetition rate. Note that the pulse energy is automatically reduced when the repetition rate is increased, because the total energy, which is determined by the pump current, is maintained the same. Under the given pump current, i.e., pump energy, the more pulses are produced, the less energy each pulse will have.

From the above descriptions, it can be seen that the term "laser blanking" in this disclosure does not require the laser pulses to be blocked; it only requires the absence of laser pulses with pulse energies at or above the photodisruption threshold (at the location the pulses are delivered to the tissue). This laser blanking technique may be referred to as "tissue cutting blanking" to emphasize the blanking effect on tissue cutting. Of course, "laser blanking" can also be accomplished by completely blocking the laser pulses, e.g., by using an acoustic-optical modulator.

In some embodiments, the overall lenticular incision procedure is performed in the following steps:
1. Calculate the radius of curvature of the lenticule based on the amount of optical correction, e.g., using Equation (3) for a myopic correction.
2. Select the diameter for the lenticular incision to be extracted.
3. Select laser and optical system parameters, including the laser blanking parameters.
4. Perform bottom surface dissection. In doing so, the fast scan line is preferably kept tangential to the parallels of latitude, and the trajectory of the slow sweep drawn by X, Y, and Z scanning devices moves along the meridians of longitude near south pole in a sequence of 1A to 1B (first sweep of lenticular cut), 2A to 2B (second sweep of lenticular cut), 3A to 3B (third sweep of lenticular cut), and so on, applying periodic laser blanking in the central area for each sweep, until the full bottom dissection surface is generated.
5. Perform the lenticule side (edge) incision.
6. Perform the top surface dissection in a similar manner as the bottom dissection is done.
7. Perform the entry incision.

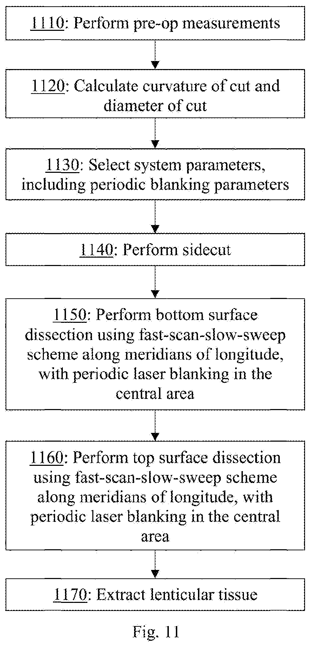

FIG. 11 illustrates a process of the laser system 10 according to an embodiment. The laser system 10 may start a surgical procedure performing pre-operation measurements (Action Block 1110). For example, in an ophthalmologic surgery for myopic correction, the myopic diopter is determined, the reference depth position is determined, and so on. The laser system 10 calculates the radius of curvature based on the amount of correction, e.g., the myopic correction determined in pre-operation measurements, as shown, for example, in equations (2) and (3) above, and calculates the diameter of the incision, as shown by D_{CUT} in FIG. 7 (Action Block 1120). D_{CUT} is equal to or greater than the diameter of the to-be-extracted lenticule (DL in FIG. 7). The system select various laser and optical system parameters, including laser blanking parameters (Action Block 1130).

The laser system 10 first performs side incision to provide a vent for gas that can be produced in the lenticular surface dissections, and for tissue extraction later on (Action Block 1140). The laser system 10 then performs the bottom lenticular surface dissection (Action Block 1150) and the top lenticular surface dissection (Action Block 1160). The bottom and top lenticular surface dissection are performed using a fast-scan-slow-sweep scheme along the meridians of longitude, with periodic laser blanking in the central area, as described above. The lenticular tissue is then extracted (Action Block 1170). Alternatively, the side incision may be performed after the bottom and top lenticular surface dissections.

The above described embodiments solve the problem of redundant energy deposit near the central area by reducing the number of laser pulses delivered in the central area.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate embodiments of the invention and does not pose a limitation on the scope of the invention unless otherwise claimed. No language in the specification should be construed as indicating any non-claimed element as essential to the practice of the invention.

While certain illustrated embodiments of this disclosure have been shown and described in an exemplary form with a certain degree of particularity, those skilled in the art will understand that the embodiments are provided by way of example only, and that various variations can be made without departing from the scope of the invention. Thus, it is intended that this disclosure cover all modifications, alternative constructions, changes, substitutions, variations, as well as the combinations and arrangements of parts, structures, and steps that come within the scope of the invention as generally expressed by the following claims.

## Claims

1. An ophthalmic surgical laser system comprising:
a laser source (14) configured to generate a pulsed laser beam comprising a plurality of laser pulses;
a laser delivery system configured to deliver the pulsed laser beam to a target tissue in a subject's eye;
a high frequency scanner (21, 25) configured to scan the pulsed laser beam back and forth at a predefined frequency;
an XY-scanner (28) configured to deflect the pulsed laser beam, the XY-scanner being separate from the high frequency scanner;
a Z-scanner (27) configured to modify a depth of a focus of the pulsed laser beam; and
a controller (22) configured to control the laser source, the high frequency scanner, the XY-scanner and the Z-scanner to successively form a plurality of sweeps (1004) which collectively form at least one lenticular incision of a lens in the subject's eye, the lens having a curved surface that defines an apex and a Z axis passing through the apex, wherein each sweep is formed by:
controlling the high frequency scanner to deflect the pulsed laser beam to form a scan line, the scan line being a straight line having a predefined length and being tangential to a parallel of latitude (510) of the lens, the parallel of latitude being a circle on the surface of the lens that is perpendicular to the Z axis and has a defined distance to the apex,
controlling the XY-scanner and the Z-scanner to move the scan line along a meridian of longitude (520) of the lens, the meridian of longitude being a curve that passes through the apex and has a defined angular position around the Z axis, and
controlling the laser source to periodically blank the pulsed laser beam when the scan line is located within a central area (1003) of the lens by periodically blanking the pulsed laser beam with a duty cycle of 5-95% and a period of 1.0-50.0 ms,
wherein the plurality of sweeps are successively formed one after another along the respective meridians of longitude which are different from one another.

2. The ophthalmic surgical laser system of claim 1, wherein the step of controlling the laser source to periodically blank the pulsed laser beam includes periodically reducing a pulse energy of the laser pulses to a value below a photodisruption threshold of the target tissue.

3. The ophthalmic surgical laser system of claim 1, wherein the step of controlling the laser source to periodically blank the pulsed laser beam includes periodically increasing a repetition rate of the laser pulses and reducing a pulse energy of the laser pulses to a value below a photodisruption threshold of the target tissue.

4. The ophthalmic surgical laser system of claim 1, wherein the step of controlling the laser source to periodically blank the pulsed laser beam includes blanking the pulsed laser beam for 1 to 95% of a time when the scan line is located within the central area of the lens.

5. The ophthalmic surgical laser system of claim 1, wherein the central area of the lens has a radius of 0.25-2.5 mm.

6. The ophthalmic surgical laser system of claim 1, wherein the controller is configured to move the scan line along the meridian of longitude of the lens at a speed of 10-100 mm/s in the central area.

7. The ophthalmic surgical laser system of claim 1, wherein the high frequency scanner is a resonant scanner with a scanning frequency between 0.5 kHz and 20 kHz, and the predetermined length of the scan lines is between 0.2 mm and 1.2 mm.

8. The ophthalmic surgical laser system of claim 1, further comprising a prism (23) disposed to receive scanned pulsed laser beam from the high frequency scanner, and wherein the controller is configured to rotate the prism to rotate an orientation of the scan line between successive sweeps.

9. The ophthalmic surgical laser system of claim 1, wherein the at least one lenticular incision includes a top lenticular incision and a bottom lenticular incision, wherein the curved surface is a top surface of the lens corresponding to the top lenticular incision, the lens further including a bottom surface corresponding to the bottom lenticular incision and defining another apex, and wherein the scan line for each of the sweeps forming the top lenticular incision is moved over the top surface of the lens through the apex of the top surface of the lens, and the scan line for each of the sweeps forming the bottom lenticular incision is moved over the bottom surface of the lens through the other apex of the bottom surface of the lens.

10. A computer program product configured to be operated on a controller in a laser surgical system, wherein the laser surgical system comprises:
a laser source (14,) configured to generate a pulsed laser beam comprising a plurality of laser pulses;
a laser delivery system configured to deliver the pulsed laser beam to a target tissue in a subject's eye;
a high frequency scanner (21, 25) configured to scan the pulsed laser beam back and forth at a predefined frequency;
an XY-scanner (28) configured to deflect the pulsed laser beam, the XY-scanner being separate from the high frequency scanner;
a Z-scanner (27) configured to modify a depth of a focus of the pulsed laser beam; and
the controller (22), wherein the computer program product causes the controller to control the laser source, the high frequency scanner, the XY-scanner and the Z-scanner to successively form a plurality of sweeps (1004) which collectively form at least one lenticular incision of a lens in the subject's eye, the lens having a curved surface that defines an apex and a Z axis passing through the apex, including forming each sweep by:
controlling the high frequency scanner to deflect the pulsed laser beam to form a scan line, the scan line being a straight line having a predefined length and being tangential to a parallel of latitude of the lens, the parallel of latitude being a circle on the surface of the lens that is perpendicular to the Z axis and has a defined distance to the apex,
controlling the XY-scanner and the Z-scanner to move the scan line along a meridian of longitude of the lens, the meridian of longitude being a curve that passes through the apex and has a defined angular position around the Z axis, and
controlling the laser source to periodically blank the pulsed laser beam when the scan line is located within a central area (1003) of the lens by periodically blanking the pulsed laser beam with a duty cycle of 5-95% and a period of 1.0-50.0 ms,
wherein the plurality of sweeps are successively formed one after another along the respective meridians of longitude which are different from one another.

11. The computer program product of claim 10, wherein the step of controlling the laser source to periodically blank the pulsed laser beam includes periodically reducing a pulse energy of the laser pulses to a value below a photodisruption threshold of the target tissue, or wherein the step of controlling the laser source to periodically blank the pulsed laser beam includes periodically increasing a repetition rate of the laser pulses and reducing a pulse energy of the laser pulses to a value below a photodisruption threshold of the target tissue.

12. The computer program product of claim 10, wherein the step of controlling the laser source to periodically blank the pulsed laser beam includes blanking the pulsed laser beam for 1 to 95% of time when the scan line is located within the central area of the lens.

13. The computer program product of claim 10, wherein the central area of the lens has a radius of 0.25-2.5 mm.

14. The computer program product of claim 10, wherein the laser surgical system further comprises a prism (23) disposed to receive scanned pulsed laser beam from the high frequency scanner, and the computer program product further causes the controller to control the prism to rotate an orientation of the scan line between successive sweeps.

15. The computer program product of claim 10, wherein the at least one lenticular incision includes a top lenticular incision and a bottom lenticular incision, wherein the curved surface is a top surface of the lens corresponding to the top lenticular incision, the lens further including a bottom surface corresponding to the bottom lenticular incision and defining another apex, and wherein the scan line for each of the sweeps forming the top lenticular incision is moved over the top surface of the lens through the apex of the top surface of the lens, and the scan line for each of the sweeps forming the bottom lenticular incision is moved over the bottom surface of the lens through the other apex of the bottom surface of the lens.

## Patentansprüche

1. Ophthalmisches chirurgisches Lasersystem, umfassend:
eine Laserquelle (14), die konfiguriert ist, um einen gepulsten Laserstrahl zu erzeugen, umfassend eine Vielzahl von Laserpulsen;
ein Laserabgabesystem, das konfiguriert ist, um den gepulsten Laserstrahl an ein Zielgewebe in einem Auge eines Subjekts abzugeben;
einen Hochfrequenzscanner (21, 25), der konfiguriert ist, um den gepulsten Laserstrahl mit einer vordefinierten Frequenz hin und her zu scannen;
einen XY-Scanner (28), der konfiguriert ist, um den gepulsten Laserstrahl abzulenken, wobei der XY-Scanner von dem Hochfrequenzscanner getrennt ist;
einen Z-Scanner (27), der konfiguriert ist, um eine Tiefe eines Fokus des gepulsten Laserstrahls zu ändern; und
eine Steuerung (22), die konfiguriert ist, um die Laserquelle, den Hochfrequenzscanner, den XY-Scanner und den Z-Scanner zu steuern, um aufeinanderfolgend eine Vielzahl von Durchläufen (1004) auszubilden, die gemeinsam mindestens einen linsenförmigen Einschnitt einer Linse in dem Auge des Subjekts ausbilden, wobei die Linse eine gekrümmte Oberfläche aufweist, die einen Scheitelpunkt und eine durch den Scheitelpunkt verlaufende Z-Achse definiert, wobei jeder Durchlauf ausgebildet ist durch:
Steuern des Hochfrequenzscanners, um den gepulsten Laserstrahl abzulenken, um eine Scanlinie auszubilden, wobei die Scanlinie eine gerade Linie ist, die eine vordefinierte Länge aufweist und tangential zu einem Breitenparallel (510) der Linse verläuft, wobei das Breitenparallel ein Kreis auf der Oberfläche der Linse ist, der senkrecht zu der Z-Achse steht und einen definierten Abstand zu dem Scheitelpunkt aufweist,
Steuern des XY-Scanners und des Z-Scanners, um die Scanlinie entlang eines Längen-(520)meridians der Linse zu bewegen, wobei der Längenmeridian eine Kurve ist, die durch den Scheitelpunkt verläuft und eine definierte Winkelposition um die Z-Achse herum aufweist, und
Steuern der Laserquelle, um den gepulsten Laserstrahl periodisch auszutasten, wenn sich die Scanlinie innerhalb eines zentralen Bereichs (1003) der Linse befindet, durch periodisches Austasten des gepulsten Laserstrahls mit einem Tastverhältnis von 5 bis 95 % und einer Periodendauer von 1,0 bis 50,0 ms,
wobei die Vielzahl von Durchläufen nacheinander entlang der jeweiligen Längenmeridiane ausgebildet wird, die sich voneinander unterscheiden.

2. Ophthalmisches, chirurgisches Lasersystem nach Anspruch 1, wobei der Schritt des Steuerns der Laserquelle, um den gepulsten Laserstrahl periodisch auszutasten, das periodische Reduzieren einer Pulsenergie der Laserpulse auf einen Wert unterhalb einer Photodisruptionsschwelle des Zielgewebes einschließt.

3. Ophthalmisches chirurgisches Lasersystem nach Anspruch 1, wobei der Schritt des Steuerns der Laserquelle, um den gepulsten Laserstrahl periodisch auszutasten, das periodische Erhöhen einer Wiederholungsrate der Laserpulse und das Reduzieren einer Pulsenergie der Laserpulse auf einen Wert unterhalb einer Photodisruptionsschwelle des Zielgewebes einschließt.

4. Ophthalmisches chirurgisches Lasersystem nach Anspruch 1, wobei der Schritt des Steuerns der Laserquelle, um den gepulsten Laserstrahl periodisch auszutasten, das Austasten des gepulsten Laserstrahls für 1 bis 95 % einer Zeit einschließt, wenn sich die Scanlinie innerhalb des zentralen Bereichs der Linse befindet.

5. Ophthalmisches chirurgisches Lasersystem nach Anspruch 1, wobei der zentrale Bereich der Linse einen Radius von 0,25 b 2,5 mm aufweist.

6. Ophthalmisches chirurgisches Lasersystem nach Anspruch 1, wobei die Steuerung konfiguriert ist, um die Scanlinie entlang des Längenmeridians der Linse mit einer Geschwindigkeit von 10 bis 100 mm/s in dem zentralen Bereich zu bewegen.

7. Ophthalmisches chirurgisches Lasersystem nach Anspruch 1, wobei der Hochfrequenzscanner ein Resonanzscanner mit einer Scanfrequenz zwischen 0,5 kHz und 20 kHz ist und die vorbestimmte Länge der Scanlinien zwischen 0,2 mm und 1,2 mm liegt.

8. Ophthalmisches chirurgisches Lasersystem nach Anspruch 1, ferner umfassend ein Prisma (23), das angeordnet ist, um einen gescannten gepulsten Laserstrahl von dem Hochfrequenzscanner zu empfangen, und wobei die Steuerung konfiguriert ist, um das Prisma zu drehen, um eine Ausrichtung der Scanlinie zwischen aufeinanderfolgenden Durchläufen zu drehen.

9. Ophthalmisches chirurgisches Lasersystem nach Anspruch 1, wobei der mindestens eine linsenförmige Einschnitt einen oberen linsenförmigen Einschnitt und einen unteren linsenförmigen Einschnitt einschließt, wobei die gekrümmte Oberfläche eine obere Oberfläche der Linse ist, die dem oberen linsenförmigen Einschnitt entspricht, wobei die Linse ferner eine untere Oberfläche einschließt, die dem unteren linsenförmigen Einschnitt entspricht und einen weiteren Scheitelpunkt definiert, und wobei die Scanlinie für jeden der Durchläufe, die den oberen linsenförmigen Einschnitt ausbilden, über die obere Oberfläche der Linse durch den Scheitelpunkt der oberen Oberfläche der Linse bewegt wird, und die Scanlinie für jeden der Durchläufe, die den unteren linsenförmigen Einschnitt ausbilden, über die untere Oberfläche der Linse durch den anderen Scheitelpunkt der unteren Oberfläche der Linse bewegt wird.

10. Computerprogrammprodukt, das konfiguriert ist, um auf einer Steuerung in einem laserchirurgischen System betrieben zu werden, wobei das laserchirurgische System umfasst:
eine Laserquelle (14), die konfiguriert ist, um einen gepulsten Laserstrahl zu erzeugen, der eine Vielzahl von Laserpulsen umfasst;
ein Laserabgabesystem, das konfiguriert ist, um den gepulsten Laserstrahl an ein Zielgewebe in einem Auge eines Subjekts abzugeben;
einen Hochfrequenzscanner (21, 25), der konfiguriert ist, um den gepulsten Laserstrahl mit einer vordefinierten Frequenz hin und her zu scannen;
einen XY-Scanner (28), der konfiguriert ist, um den gepulsten Laserstrahl abzulenken, wobei der XY-Scanner von dem Hochfrequenzscanner getrennt ist;
einen Z-Scanner (27), der konfiguriert ist, um eine Tiefe eines Fokus des gepulsten Laserstrahls zu ändern; und
die Steuerung (22), wobei das Computerprogrammprodukt die Steuerung veranlasst, die Laserquelle, den Hochfrequenzscanner, den XY-Scanner und den Z-Scanner zu steuern, um nacheinander eine Vielzahl von Durchläufen (1004) auszubilden, die gemeinsam mindestens einen linsenförmigen Einschnitt einer Linse in dem Auge des Subjekts ausbilden, wobei die Linse eine gekrümmte Oberfläche aufweist, die einen Scheitelpunkt und eine durch den Scheitelpunkt verlaufende Z-Achse definiert, einschließlich Ausbilden jedes Durchlaufs durch:
Steuern des Hochfrequenzscanners, um den gepulsten Laserstrahl abzulenken, um eine Scanlinie auszubilden, wobei die Scanlinie eine gerade Linie ist, die eine vordefinierten Länge aufweist und tangential zu einem Breitenparallel der Linse verläuft, wobei das Breitenparallel ein Kreis auf der Oberfläche der Linse ist, der senkrecht zu der Z-Achse steht und einen definierten Abstand zu dem Scheitelpunkt aufweist,
Steuern des XY-Scanners und des Z-Scanners, um die Scanlinie entlang eines Längenmeridians der Linse zu bewegen, wobei der Längenmeridian eine Kurve ist, die durch den Scheitelpunkt verläuft und eine definierte Winkelposition um die Z-Achse herum aufweist, und
Steuern der Laserquelle, um den gepulsten Laserstrahl periodisch auszutasten, wenn sich die Scanlinie innerhalb eines zentralen Bereichs (1003) der Linse befindet, durch periodisches Austasten des gepulsten Laserstrahls mit einem Tastverhältnis von 5 bis 95 % und einer Periodendauer von 1,0 bis 50,0 ms,
wobei die Vielzahl von Durchläufen nacheinander entlang der jeweiligen Längenmeridiane ausgebildet wird, die sich voneinander unterscheiden.

11. Computerprogrammprodukt nach Anspruch 10, wobei der Schritt des Steuerns der Laserquelle, um den gepulsten Laserstrahl periodisch auszutasten, das periodische Reduzieren einer Pulsenergie der Laserpulse auf einen Wert unterhalb einer Photodisruptionsschwelle des Zielgewebes einschließt, oder wobei der Schritt des Steuerns der Laserquelle, um den gepulsten Laserstrahl periodisch auszutasten, das periodische Erhöhen einer Wiederholungsrate der Laserpulse und Reduzieren einer Pulsenergie der Laserpulse auf einen Wert unterhalb einer Photodisruptionsschwelle des Zielgewebes einschließt.

12. Computerprogrammprodukt nach Anspruch 10, wobei der Schritt des Steuerns der Laserquelle, um den gepulsten Laserstrahl periodisch auszutasten, das Austasten des gepulsten Laserstrahls für 1 bis 95 % der Zeit einschließt, wenn sich die Scanlinie innerhalb des zentralen Bereichs der Linse befindet.

13. Computerprogrammprodukt nach Anspruch 10, wobei der zentrale Bereich der Linse einen Radius von 0,25 bis 2,5 mm aufweist.

14. Computerprogrammprodukt nach Anspruch 10, wobei das Laserchirurgiesystem ferner ein Prisma (23) umfasst, das angeordnet ist, um einen gescannten gepulsten Laserstrahl von dem Hochfrequenzscanner zu empfangen, und das Computerprogrammprodukt ferner die Steuerung veranlasst, das Prisma zu steuern, um eine Ausrichtung der Scanlinie zwischen aufeinanderfolgenden Durchläufen zu drehen.

15. Computerprogrammprodukt nach Anspruch 10, wobei der mindestens eine linsenförmige Einschnitt einen oberen linsenförmigen Einschnitt und einen unteren linsenförmigen Einschnitt einschließt, wobei die gekrümmte Oberfläche eine obere Oberfläche der Linse ist, die dem oberen linsenförmigen Einschnitt entspricht, wobei die Linse ferner eine untere Oberfläche einschließt, die dem unteren linsenförmigen Einschnitt entspricht und einen weiteren Scheitelpunkt definiert, und wobei die Scanlinie für jeden der Durchläufe, die den oberen linsenförmigen Einschnitt ausbilden, über die obere Oberfläche der Linse durch den Scheitelpunkt der oberen Oberfläche der Linse bewegt wird, und die Scanlinie für jeden der Durchläufe, die den unteren linsenförmigen Einschnitt ausbilden, über die untere Oberfläche der Linse durch den anderen Scheitelpunkt der unteren Oberfläche der Linse bewegt wird.

## Revendications

1. Système laser chirurgical ophtalmique comprenant :
une source laser (14) configurée pour générer un faisceau laser pulsé comprenant une pluralité d'impulsions laser ;
un système de délivrance laser configuré pour délivrer le faisceau laser pulsé vers un tissu cible dans l'œil d'un sujet ;
un dispositif de balayage haute fréquence (21, 25) configuré pour balayer le faisceau laser pulsé en va-et-vient à une fréquence prédéfinie ;
un dispositif de balayage XY (28) configuré pour dévier le faisceau laser pulsé, le dispositif de balayage XY étant séparé du dispositif de balayage haute fréquence ;
un dispositif de balayage Z (27) configuré pour modifier la profondeur d'un foyer du faisceau laser pulsé ; et
une unité de commande (22) configurée pour commander la source laser, le dispositif de balayage haute fréquence, le dispositif de balayage XY et le dispositif de balayage Z pour former successivement une pluralité de cycles d'acquisition (1004) qui forment collectivement au moins une incision lenticulaire d'un cristallin dans l'œil du sujet, le cristallin ayant une surface courbée qui définit un apex et un axe Z passant à travers l'apex, dans lequel chaque cycle d'acquisition est formé par :
la commande du dispositif de balayage haute fréquence pour dévier le faisceau laser pulsé pour former une ligne de balayage, la ligne de balayage étant une ligne droite ayant une longueur prédéfinie et étant tangente à une parallèle de latitude (510) du cristallin, la parallèle de latitude étant un cercle sur la surface du cristallin qui est perpendiculaire à l'axe Z et a une distance définie par rapport à l'apex,
la commande du dispositif de balayage XY et du dispositif de balayage Z pour déplacer la ligne de balayage le long d'un méridien de longitude (520) du cristallin, le méridien de longitude étant une courbe qui passe à travers l'apex et a une position angulaire définie autour de l'axe Z, et
la commande de la source laser pour occulter périodiquement le faisceau laser pulsé lorsque la ligne de balayage est localisée au sein d'une zone centrale (1003) du cristallin en occultant périodiquement le faisceau laser pulsé avec un cycle de service de 5 à 95 % et une période de 1,0 à 50,0 ms,
dans lequel la pluralité de cycles d'acquisition sont successivement formés les uns après les autres le long des méridiens de longitude respectifs qui sont différents les uns des autres.

2. Système laser chirurgical ophtalmique selon la revendication 1, dans lequel l'étape de commande de la source laser pour occulter périodiquement le faisceau laser pulsé comporte la réduction périodique d'une énergie d'impulsion des impulsions laser à une valeur inférieure à un seuil de photodisruption du tissu cible.

3. Système laser chirurgical ophtalmique selon la revendication 1, dans lequel l'étape de commande de la source laser pour occulter périodiquement le faisceau laser pulsé comporte l'augmentation périodique d'une fréquence de répétition des impulsions laser et la réduction d'une énergie d'impulsion des impulsions laser à une valeur inférieure à un seuil de photodisruption du tissu cible.

4. Système laser chirurgical ophtalmique selon la revendication 1, dans lequel l'étape de commande de la source laser pour occulter périodiquement le faisceau laser pulsé comporte l'occultation du faisceau laser pulsé pendant 1 à 95 % d'un temps où la ligne de balayage est localisée au sein de la zone centrale du cristallin.

5. Système laser chirurgical ophtalmique selon la revendication 1, dans lequel la zone centrale du cristallin a un rayon de 0,25 à 2,5 mm.

6. Système laser chirurgical ophtalmique selon la revendication 1, dans lequel l'unité de commande est configurée pour déplacer la ligne de balayage le long du méridien de longitude du cristallin à une vitesse de 10 à 100 mm/s dans la zone centrale.

7. Système laser chirurgical ophtalmique selon la revendication 1, dans lequel le dispositif de balayage haute fréquence est un dispositif de balayage à résonance avec une fréquence de balayage entre 0,5 kHz et 20 kHz, et la longueur prédéterminée des lignes de balayage est comprise entre 0,2 mm et 1,2 mm.

8. Système laser chirurgical ophtalmique selon la revendication 1, comprenant en outre un prisme (23) disposé pour recevoir le faisceau laser pulsé balayé provenant du dispositif de balayage haute fréquence, et dans lequel l'unité de commande est configurée pour mettre en rotation le prisme pour mettre en rotation une orientation de la ligne de cycles d'acquisition entre des cycles d'acquisition successifs.

9. Système laser chirurgical ophtalmique selon la revendication 1, dans lequel l'au moins une incision lenticulaire comporte une incision lenticulaire supérieure et une incision lenticulaire inférieure, dans lequel la surface incurvée est une surface supérieure du cristallin correspondant à l'incision lenticulaire supérieure, le cristallin comportant en outre une surface inférieure correspondant à l'incision lenticulaire inférieure et définissant un autre apex, et dans lequel la ligne de balayage pour chacun des cycles d'acquisition formant l'incision lenticulaire supérieure est déplacée sur la surface supérieure du cristallin à travers l'apex de la surface supérieure du cristallin, et la ligne de balayage pour chacun des cycles d'acquisition formant l'incision lenticulaire inférieure est déplacée sur la surface inférieure du cristallin à travers l'autre apex de la surface inférieure du cristallin.

10. Produit programme informatique configuré pour être mis en fonctionnement sur une unité de commande dans un système chirurgical laser, dans lequel le système chirurgical laser comprend :
une source laser (14), configurée pour générer un faisceau laser pulsé comprenant une pluralité d'impulsions laser ;
un système de délivrance laser configuré pour délivrer le faisceau laser pulsé vers un tissu cible dans l'œil d'un sujet ;
un dispositif de balayage haute fréquence (21, 25) configuré pour balayer le faisceau laser pulsé en va-et-vient à une fréquence prédéfinie ;
un dispositif de balayage XY (28) configuré pour dévier le faisceau laser pulsé, le dispositif de balayage XY étant séparé du dispositif de balayage haute fréquence ;
un dispositif de balayage Z (27) configuré pour modifier la profondeur d'un foyer du faisceau laser pulsé ; et
l'unité de commande (22), dans lequel le produit programme informatique amène l'unité de commande à commander la source laser, le dispositif de balayage haute fréquence, le dispositif de balayage XY et le dispositif de balayage Z pour former successivement une pluralité de cycles d'acquisition (1004) qui forment collectivement au moins une incision lenticulaire d'un cristallin dans l'œil du sujet, le cristallin ayant une surface incurvée qui définit un apex et un axe Z passant à travers l'apex, comportant la formation de chaque cycle d'acquisition par :
la commande du dispositif de balayage haute fréquence pour dévier le faisceau laser pulsé pour former une ligne de balayage, la ligne de balayage étant une ligne droite ayant une longueur prédéfinie et étant tangente à une parallèle de latitude du cristallin, la parallèle de latitude étant un cercle sur la surface du cristallin qui est perpendiculaire à l'axe Z et a une distance définie par rapport à l'apex,
la commande du dispositif de balayage XY et du dispositif de balayage Z pour déplacer la ligne de balayage le long d'un méridien de longitude du cristallin, le méridien de longitude étant une courbe qui passe à travers l'apex et a une position angulaire définie autour de l'axe Z, et
la commande de la source laser pour occulter périodiquement le faisceau laser pulsé lorsque la ligne de balayage est localisée au sein d'une zone centrale (1003) du cristallin en occultant périodiquement le faisceau laser pulsé avec un cycle de service de 5 à 95 % et une période de 1,0 à 50,0 ms,
dans lequel la pluralité de cycles d'acquisition sont successivement formés les uns après les autres le long des méridiens de longitude respectifs qui sont différents les uns des autres.

11. Produit programme informatique selon la revendication 10, dans lequel l'étape de commande de la source laser pour occulter périodiquement le faisceau laser pulsé comporte la réduction périodique d'une énergie d'impulsion des impulsions laser à une valeur inférieure à un seuil de photodisruption du tissu cible, ou dans lequel l'étape de commande de la source laser pour occulter périodiquement le faisceau laser pulsé comporte l'augmentation périodique d'une fréquence de répétition des impulsions laser et la réduction d'une énergie d'impulsion des impulsions laser à une valeur inférieure à un seuil de photodisruption du tissu cible.

12. Produit programme informatique selon la revendication 10, dans lequel l'étape de commande de la source laser pour occulter périodiquement le faisceau laser pulsé comporte l'occultation du faisceau laser pulsé pendant 1 à 95 % d'un temps où la ligne de balayage est localisée au sein de la zone centrale du cristallin.

13. Produit programme informatique selon la revendication 10, dans lequel la zone centrale du cristallin a un rayon de 0,25 à 2,5 mm.

14. Produit programme informatique selon la revendication 10, dans lequel le système chirurgical laser comprend en outre un prisme (23) disposé pour recevoir un faisceau laser pulsé balayé provenant du dispositif de balayage haute fréquence, et le produit programme informatique amène en outre l'unité de commande à commander le prisme pour mettre en rotation une orientation de la ligne de balayage entre des cycles d'acquisition successifs.

15. Système laser chirurgical ophtalmique selon la revendication 10, dans lequel l'au moins une incision lenticulaire comporte une incision lenticulaire supérieure et une incision lenticulaire inférieure, dans lequel la surface incurvée est une surface supérieure du cristallin correspondant à l'incision lenticulaire supérieure, le cristallin comportant en outre une surface inférieure correspondant à l'incision lenticulaire inférieure et définissant un autre apex, et dans lequel la ligne de balayage pour chacun des cycles d'acquisition formant l'incision lenticulaire supérieure est déplacée sur la surface supérieure du cristallin à travers l'apex de la surface supérieure du cristallin, et la ligne de balayage pour chacun des cycles d'acquisition formant l'incision lenticulaire inférieure est déplacée sur la surface inférieure du cristallin à travers l'autre apex de la surface inférieure du cristallin.
